# EUROPÄISCHE PATENTSCHRIFT

(11) **EP 1 598 331 B1**
(45) Veröffentlichungstag und Bekanntmachung des Hinweises auf die Patenterteilung: **15.07.2009**
(21) Anmeldenummer: 05010222.7
(22) Anmeldetag: 11.05.2005
(51) Int. Cl.: C07C 51/60, C07C 63/22, C07C 231/02

(54) **Verfahren zur Herstellung von Phthalsäuredichlorid**
Process for the preparation of phthalic acid dichloride
Procédé pour la préparation de l' anhydride phtalique

(30) Priorität: 17.05.2004 DE 102004024807
(43) Veröffentlichungstag der Anmeldung: 23.11.2005
(73) Patentinhaber: Saltigo GmbH, 40764 Langenfeld (DE)
(72) Erfinder: Job, Andreas, Dr., 50858 Köln (DE)
(74) Vertreter: Wichmann, Birgid

(56) Entgegenhaltungen:
- EP-A- 0 050 779
- EP-A- 0 340 706
- EP-A- 0 795 546
- WO-A-20/04022520
- US-A- 3 318 950
- US-A- 3 810 940
- DATABASE CA [Online] CHEMICAL ABSTRACTS SERVICE, COLUMBUS, OHIO, US; "Carboxylic acid chlorides" XP002341528 gefunden im STN Database accession no. 1982:19845 & JP 56 103131 A2 (MITSUI TOATSU CHEMICALS, INC., JAPAN) 18. August 1981 (1981-08-18)

## Beschreibung

Die Erfindung betrifft ein neues Verfahren zur Herstellung von Phthalsäuredichlorid (Benzol-1,2-dicarbonsäure-dichlorid) aus Phthalsäureanhydrid.

Üblicherweise wird Phthalsäuredichlorid aus Phthalsäureanhydrid durch Umsetzung mit verschiedenen Mitteln zur Einführung von Chlor "(Chlorierungsmitteln") hergestellt.

Gemäß US-A-2,051,096 erhält man Phthalsäuredichlorid durch Umsetzung von Phthalsäureanhydrid mit einer Substanz, die mindestens zwei Chlor-Atome an einem Kohlenstoff-Atom trägt, in Gegenwart eines Katalysators wie Zinkchlorid, Aluminiumchlorid oder Eisenchlorid. Bevorzugt wird unter Einsatz von Trichlormethan oder Tetrachlormethan gearbeitet. Diese Umsetzung erfordert jedoch sehr hohe Temperaturen im Bereich von 250 bis 300°C. Außerdem sind Trichlormethan bzw. Tetrachlormethan heute für industrielle Belange sehr problematische Reaktionskomponenten.

In J. Am. Chem. Soc. 1937, 59, 206-208 wird die Herstellung von Phthalsäuredichlorid durch Umsetzung von Phthalsäureanhydrid mit Thionylchlorid oder Benzotrichlorid in Gegenwart von wasserfreiem Zinkchlorid beschrieben. Auch diese Umsetzung erfordert jedoch hohe Temperaturen. Zudem sind Ausbeute und Qualität des Produktes bei dieser Methode nicht ganz zufriedenstellend. Insbesondere enthält das gewünschte Produkt Phthalsäuredichlorid noch gewisse Mengen gelösten Phthalsäureanhydrids.

Gemäß Can. J. Chem. 1970, 48, 3566-3571 kann man Phthalsäuredichlorid auch durch Umsetzung von Phthalsäureanhydrid mit Phosphor-(V)-chlorid (Phosphorpentachlorid) erhalten, allerdings ebenfalls mit einer nur unbefriedigenden Ausbeute von 54 % an dem gewünschten Produkt.

Gemäß DE-A 20 36 171 kann Phthalsäuredichlorid durch Umsetzung von Phthalsäureanhydrid mit Trichlormethylisocyaniddichlorid in Gegenwart von Eisen(III)-chlorid erhalten werden. Hierbei fällt jedoch Chlorcarbonylisocyaniddichlorid als Koppelprodukt an.

In der EP-A-0 050 779 wird die Verwendung N,N-dialkylierter Formamide als Beschleuniger beim Austausch von Hydroxylgruppen in organischen Verbindungen gegen Chlor oder Brom sowie als Katalysator für die Umsetzung von phenolischen Verbindungen mit Phosgen zu Arylchlorameisensäureestern beschrieben.

Aus der US-A-3,810,940 ist ferner die Umsetzung von intramolekularen Anhydriden mit Phosgen in einem inerten aromatischen Lösungsmittel beschrieben, wobei als Katalysator ein Carboxamid der Formel RCONR'₂ eingesetzt wird, in dem R für Wasserstoff oder eine niedere Alkylgruppe mit bevorzugt 1-4 C-Atomen steht und R' ebenfalls eine niedere Alkylgruppe mit bevorzugt 1-4 C-Atomen darstellt. Insbesondere im Falle der Herstellung von unsubstituierten cyclischen aromatischen Disäuredichloriden aus den entsprechenden intramolekularen Anhydriden wird hier ein nur unvollständiger Umsatz erzielt. Beschrieben wird explizit die Umsetzung von Phthalsäureanhydrid mit Phosgen in Chlorbenzol in Gegenwart von N,N-Dimethyl-formamid als Katalysator. Der erhaltene Umsatz ist aber nicht zufriedenstellend. Da sich aufgrund der zu niedrigen Siedepunktsdifferenz zwischen dem gewünschten Produkt Phthalsäuredichlorid und dem Ausgangsmaterial Phthalsäureanhydrid das Ausgangsmaterial nicht destillativ vom Produkt abtrennen lässt, ist aber ein möglichst hoher Umsatz für eine gute Produktqualität essentiell.

Aus der WO-A-04/022520 ist ein weiteres Verfahren zur Herstellung von Phthalsäuredichlorid durch Umsetzung von Phthalsäureanhydrid mit Phosgen in einem inerten Verdünnungsmittel in Gegenwart eines N,N-Dialkyl-formamids bekannt. Kennzeichnend für dieses Verfahren ist, dass das Formamid und/oder Phosgen kontinuierlich oder semikontinuierlich zudosiert werden. Als Substituenten der Formamide werden ganz allgemein geradkettige oder verzweigte Alkyl-Reste genannt. Als bevorzugte Reste werden geradkettiges oder verzweigtes C₁-C₁₀-Alkyl, inbesondere C₁-C₆-Alkyl genannt. Explizit genannt werden jedoch ausschließlich N,N-Dialkyl-formamide mit jeweils zwei identischen C₁-C₄-Alkyl-Substituenten am Stickstoff. Die in den Beispielen ausschließlich verwendeten Katalysatoren N,N-Dimethylformamid (DMF) und N,N-Dibutylformamid (DBF) führen in dem beschriebenen Verfahren zu unerwünschten Nebenkomponenten, den sogenannten Carbamoylchloriden. Das unter Verwendung von DMF entstehende Dimethylcarbamoylchlorid ist als krebserregende Substanz bekannt und daher hochgradig unerwünscht in industriellen Anlagen und Chemikalienzubereitungen. Das unter Verwendung von DBF entstehende und weniger giftige Dibutylcarbamoylchlorid besitzt einen ähnlichen Siedepunkt wie das herzustellende Produkt Phthalsäuredichlorid und ist somit destillativ nur unter erheblichem Aufwand von diesem zu trennen. Darüber hinaus zeigen die auf diesem Wege hergestellten Produkte eine unerwünschte, dunkel-violette Färbung.

Es bestand also die Aufgabe, ein verbessertes Verfahren zur Verfügung zu stellen, durch welches Phthalsäuredichlorid aus einem gut zugänglichen Ausgangsstoff wie Phthalsäureanhydrid unter Verwendung leicht zugänglicher Hilfsstoffe, unter vertretbarem Energieaufwand, und vor allem unter Vermeidung des Anfallens größerer Mengen unerwünschter Nebenprodukte, in sehr guten Ausbeuten erhalten werden kann.

Überraschenderweise wurde gefunden, dass ausgehend von Phthalsäureanhydrid bei Verwendung von Phosgen als Mittel zur Einführung von Chlor in Gegenwart spezieller Typen N,N-disubstituierter Formamide das gewünschte Produkt Phthalsäuredichlorid in hohen Ausbeuten und in sehr guter Reinheit erhalten werden kann, wobei insbesondere krebserregende Nebenprodukte vermieden werden können.

Gegenstand der vorliegenden Erfindung ist also ein Verfahren zur Herstellung von Phthalsäuredichlorid der Formel (I) 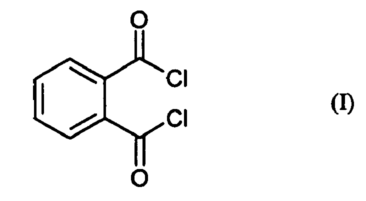 in dem man Phthalsäureanhydrid der Formel (II) 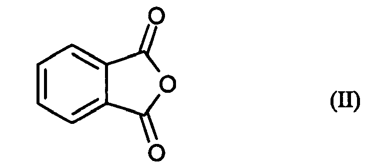 mit Phosgen in Gegenwart eines Katalysators umsetzt, wobei dieses Verfahren **dadurch gekennzeichnet** ist, dass als Katalysator ein N,N-disubstituiertes Formamid der allgemeinen Formel (III) eingesetzt wird, 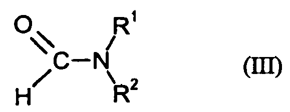 in welcher
R¹ und R² unabhängig voneinander für
- geradkettiges oder verzweigtes C₁-C₂₂-alkyl oder geradkettiges oder verzweigtes C₂-C₂₂-alkenyl,
- C₃-C₈-cycloalkyl,
- C₆-C₁₀-aryl oder
- C₇-C₁₂-arylalkyl
stehen, wobei für den Fall, dass beide Reste R¹ und R² unabhängig voneinander für geradkettiges oder verzweigtes C₁-C₂₂-alkyl oder geradkettiges oder verzweigtes C₂-C₂₂-alkenyl stehen, das Gesamtmolekulargewicht des N,N-disubstituierten Formamids mindestens 269 g/mol betragen muss.

Das beim erfindungsgemäßen Verfahren als Ausgangsstoff einzusetzende Phthalsäureanhydrid der Formel (II) ist eine bekannte, handelsübliche Synthesechemikalie.

Das als Mittel zu Einführung von Chlor verwendete Phosgen ist ebenfalls bekannt und kann in handelsüblichen Qualitäten eingesetzt werden.

Bei den im erfmdungsgemäßen Verfahren eingesetzten N,N-disubstituierten Formamiden der allgemeinen Formel (III) haben die aufgeführten Substituenten folgenden Bedeutungen:

Geradkettiges oder verzweigtes C₈-C₂₂-Alkyl steht für einen geradkettigen oder verzweigten Alkylrest mit 8 bis 22 Kohlenstoffatomen wie Octyl, Nonyl, Decyl, Undecyl, Dodecyl, Tridecyl, Tetradecyl, Pentadecyl, Hexadecyl, Heptadecyl, Octadecyl, Nonadecyl, Eicosyl oder Docosyl-Reste.

Geradkettiges oder verzweigtes C₈-C₂₂-Alkenyl steht für einen geradkettigen oder verzweigten Alkenylrest mit 8 bis 22 Kohlenstoffatomen wie z.B. Oleyl (-(CH₂)₈-CH=CH-(CH₂)₇-CH₃) oder Linoleyl (-(CH₂)₈-CH=CH-CH₂-CH=CH-(CH₂)₄-CH₃).

Sofern R¹ und R² beide für geradkettiges oder verzweigtes C₈-C₂₂-Alkyl oder geradkettiges oder verzweigtes C₈-C₂₂-Alkenyl stehen, muss das Gesamtmolekulargewicht des resultierenden N,N-disubstituierten Formamids der Formel (III) mindestens 269 g/mol betragen.

Unter Einhaltung der vorgenannten Maßgabe eines Gesamtmolekulargewichts von mindestens 269 g/mol stehen R¹ und R² in einer bevorzugten Kombination für denselben geradkettigen oder verzweigten C₈-C₁₈-Alkyl- oder-Alkenyl-Rest.

In der allgemeinen Formel (III) steht C₃-C₈-Cycloalkyl für Cyclopropyl, Cyclobutyl, Cyclopentyl, Cyclohexyl, Cycloheptyl oder Cyclooctyl. Bevorzugt seien genannt: Cyclopropyl, Cyclopentyl und Cyclohexyl.

In einer bevorzugten Kombination stehen R¹ und R² beide für unabhängig voneinander für einen C₃-C₈-Cycloalkylrest, bevorzugt stehen beide Reste R¹ und R² denselben C₃-C₈-Cycloalkylrest und inbesondere beide Reste R¹ und R² für einen Cyclopentyl oder einen Cyclohexylrest.

In der allgemeinen Formel (III) steht C₆-C₁₀-Aryl für einen aromatischen Rest mit 6 bis 10 Kohlenstoffatomen. Bevorzugte Arylreste sind Phenyl und Naphthyl.

In der allgemeinen Formel (III) steht C₇-C₁₂-Arylalkyl für einen Benzyl-, Phenylethyl-, Phenylpropyl-, Naphthylmethyl- oder einen Naphthylethyl-Rest.

Als Beispiele für N,N-disubstituierte Formamide der allgemeinen Formel (III) seien genannt:
- N,N-Dioctylformamid,
- N,N-Dinonylformamid,
- N,N-Didecylformamid,
- N,N-Diundecylformamid,
- N,N-Didodecylformamid,
- N,N-Ditridecylformamid,
- N,N-Ditetradecylformamid,
- N,N-Dipentadecylformamid,
- N,N-Dihexadecylformamid,
- N,N-Dioctadecylformamid (= N,N-distearylamid),
- N,N-Dicyclopentylformamid,
- N,N-Dicyclohexylformamid,
- N,N-Dibenzylformamid.

Die N,N-disubstituierten Formamide sind bekannte organische Synthesechemikalien bzw. Reagenzien, die käuflich erhältlich sind. Unter prinzipieller Anwendung des in der EP-A-0 050 779 beschriebenen Verfahrens ist es ferner möglich, sie durch Umsetzung des entsprechenden Amins der Formel HNR¹R² mit Ameisensäure bei erhöhter Temperatur herzustellen. Diese Umsetzung kann ohne, aber auch mit inertem Lösungsmittel durchgeführt werden. Als inerte Lösungsmittel kommen dabei die gleichen Lösungsmittel in Frage, die nachfolgend für die erfindungsgemäße Umsetzung genannt sind. Das bei der Umsetzung von Amin der Formel HNR¹R² und Ameisensäure erhaltene Reaktionsgemisch kann unmittelbar für die erfindungsgemäße Umsetzung verwendet werden, ohne dass das N,N-disubstituierte Formamid zuvor isoliert werden muss.

Das erfindungsgemäße Verfahren wird üblicherweise innerhalb eines Temperaturbereichs von 20 bis 150°C, bevorzugt von 40 bis 120°C und insbesondere von 55 bis 100°C durchgeführt.

Das erfindungsgemäße Verfahren wird im Allgemeinen unter Normaldruck durchgeführt. Es ist jedoch auch möglich, das erfindungsgemäße Verfahren unter erhöhtem oder vermindertem Druck, im Allgemeinen in einem Bereich von 0,1 bis 50 bar, bevorzugt in einem Bereich von 1 bis 10 bar durchzuführen.

Zur Durchführung des erfindungsgemäßen Verfahrens setzt man auf 1 mol Phthalsäureanhydrid der Formel (II) im Allgemeinen 1,0 mol bis 3,0 mol, vorzugsweise 1,4 mol bis 2,2 mol Phosgen ein.

Ferner setzt man bezogen auf 1 mol Phthalsäureanhydrid der Formel (II) insgesamt 0,01 bis 0,20 mol, vorzugsweise 0,02 bis 0,10 mol N,N-disubstituiertes Formamid der Formel (III) ein.

Die praktische Durchführung des erfindungsgemäßen Verfahrens kann auf verschiedenen Wegen erfolgen.

So gibt es für die Zudosierung des Phosgens und des Katalysators mehrere Varianten: In einer Variante wird das Phthalsäureanhydrid im Reaktionsgefäß vorgelegt und das Phosgen und der Katalysator jeweils unabhängig voneinander kontinuierlich oder "semi-kontinuierlich" eindosiert.

Im Sinne der vorliegenden Erfindung heißt kontinuierlich, dass die jeweilige Reaktionskomponente (Phosgen und/oder das N,N-disubstituierte Formamid) ständig gleichmäßig über die gesamte Reaktionszeit in das Reaktionsgemisch eindosiert wird.

Im Sinne der vorliegenden Erfindung heißt "semi-kontinuierlich", dass die jeweilige Reaktionskomponente (Phosgen und/oder das N,N-disubstituierte Formamid) portionsweise, auf definierte Zeitabschnitte verteilt in das Reaktionsgemisch eindosiert wird. Dabei sind die einzelnen Portionen bevorzugt gleich groß und die einzelnen Zeitabschnitte bevorzugt gleich lang.

In einer Variante wird sowohl das Phosgen als auch das N,N-disubstituierte Formamid der Formel (III) kontinuierlich eindosiert.

In einer anderen Variante wird sowohl das Phosgen als auch das N,N-disubstituierte Formamid der Formel (III) auf mehrere Portionen verteilt "semi-kontinuierlich" eindosiert.

In einer weiteren Variante wird das Phosgen kontinuierlich eindosiert, während das N,N-disubstituierte Formamid der Formel (III) auf mehrere Portionen verteilt "semi-kontinuierlich" eindosiert wird.

In einer weiteren Variante wird das Phosgen auf mehrere Portionen verteilt "semi-kontinuierlich" eindosiert, während das N,N-disubstituierte Formamid der Formel (III) kontinuierlich eindosiert wird.

Bevorzugt wird das Phthalsäureanhydrid dabei gelöst in einem inerten Lösungsmittel vorgelegt.

Als inerte Lösungsmittel kommen vor allem in Betracht: Kohlenwasserstoffe, wie Pentan, Hexan, Heptan, Oktan, Cyclohexan, Methylcyclohexan, Benzol, Toluol, Xylol, und halogenierte Kohlenwasserstoffe, wie Dichlormethan, Trichlormethan, Tetrachlormethan, Chlorbenzol oder Dichlorbenzol. Toluol und Chlorbenzol werden als inerte Lösungsmittel besonders bevorzugt. Es ist aber auch möglich, das Phthalsäureanhydrid gelöst in Phthalsäuredichlorid als Lösungsmittel einzusetzen.

Üblicherweise wird das N,N-disubstituierte Formamid der Formel (III) dabei ebenfalls gelöst in einem inerten Lösungsmittel zudosiert, wobei für das Lösungsmittel die bereits genannten in Frage kommen und bevorzugt das gleiche gewählt wird wie für das Phthalsäureanhydrid.

Möglich ist es aber auch, das N,N-disubstituierte Formamid der Formel (III) ohne Lösungsmittel zuzudosieren. Sofern es sich um einen Feststoff handelt, wird dieser zunächst aufgeschmolzen und dann als Schmelze zudosiert.

In einer weiteren Ausführungsform des erfindungsgemäßen Verfahrens wird das Phthalsäureanhydrid in einem inerten Lösungsmittel zusammen mit der gesamten Menge oder einer Teilmenge des N,N-disubstituierten Formamids der Formel (III) vorgelegt. Besonders für N,N-Dicyclohexylformamid hat es sich bewährt, die gesamte Menge zusammen mit dem Phthalsäureanhydrid im Reaktionsgefäß vorzulegen, diese Mischung dann auf die Reaktionstemperatur aufzuheizen und dann das Phosgen kontinuierlich oder "semikontinuierlich" zuzudosieren. Bewährt hat es sich aber auch, eine Teilmenge von bis zu 2 mol% des N,N-disubstituierten Formamids der Formel (III) zusammen mit dem Phthalsäureanhydrid vorzulegen, die Mischung danach auf die Reaktionstemperatur aufzuheizen und dann das Phosgen und die restliche Menge des N,N-disubstituierten Formamids der Formel (III) jeweils entweder kontinuierlich über die ganze Umsetzungszeit verteilt oder "semi-kontinuierlich" einzudosieren.

In einer weiteren Ausführungsform ist es möglich, das Phthalsäureanhydrid in aufgeschmolzener Form ohne Lösungsmittel vorzulegen und anschließend das Phosgen sowie das N,N-disubstituierte Formamid der Formel (III), letzteres ebenfalls ohne Lösungsmittel und in gegebenenfalls aufgeschmolzener Form, kontinuierlich oder semikontinuierlich zuzudosieren.

Besonders vorteilhaft ist jeweils eine Umsetzungsdauer von 5 bis 15 Stunden, die je nach Ansatzgröße entsprechend variiert werden kann.

Besonders vorteilhaft ist es dabei, alle 15 bis 90 Minuten eine Zudosierung des N,N-disubstituierten Formamids der Formel (III) vorzunehmen und das Phosgen kontinuierlich oder ""semi-kontinuierlich" einzudosieren.

Nach Ende der Zugabe von Phosgen und N,N-disubstituiertem Formamid der Formel (III) wird die Reaktionsmischung vorteilhafterweise noch 1 bis 3 Stunden bei der angegebenen Reaktionstemperatur gehalten und anschließend durch Destillation unter vermindertem Druck aufgearbeitet. Nach dieser Destillation wird das Phthalsäuredichlorid in hoher Ausbeute und in sehr guter Qualität erhalten. Insbesondere weist das Produkt nur noch einen geringen Anteil nicht umgesetzten Phthalsäureanhydrids auf, bevorzugt liegt dieser Anteil bei weniger als 3 Gew.%. Als besonderer Vorteil gegenüber den Verfahren des Standes der Technik ist ferner hervorzuheben, dass die erfindungsgemäß verwendeten N,N-disubstituierten Formamide bzw. die daraus resultierenden Koppelprodukte aufgrund des wesentlich höheren Siedepunktes bei der Destillation im Sumpf verbleiben. Zudem ist das erhaltene Produkt in allen Fällen farblos, während gemäß dem Verfahren des Standes der Technik bei zwar sehr guten Ausbeuten nur violett gefärbtes und damit ohne weitere aufwändige Aufarbeitung nicht verwendbares Produkt erhalten wird.

### Herstellungsbeispiele

In allen Beispielen wird Phthalsäureanhydrid mit einem Gehalt von mindestens 99% eingesetzt.

### Beispiel 1 (erfindungsgemäß; N-Methyl-stearylformamid als Katalysator)

523,7 g (3,5 Mol) Phthalsäureanhydrid und 5,5 g (0,02 mol) N-Methyl-stearylformamid als Katalysator werden in 2203 ml Toluol vorgelegt, und die Mischung wird auf 75°C erwärmt. Bei dieser Temperatur werden über 6 Stunden 586,1 g (5,93 mol) Phosgen mit einer Einleitgeschwindigkeit von 97,7 g Phosgen pro Stunde und gleichzeitig 59,97 g N-Methylstearylformamid gelöst in 92,1 ml Toluol kontinuierlich (d.h. 10 g N-Methylstearylformamid 100%ig pro Stunde) zugegeben. Nach Ende der Dosierung von Phosgen und Katalysator wird noch 1,5 Stunden bei 75°C nachgerührt.

Zur Entfernung überschüssigen Phosgens werden bei 55°C und 60 mbar 2150,7 g Destillat abgenommen.

Als dunkles Öl bleibt das Rohprodukt zurück. Es enthält 86,6 Gew.% Phthalsäuredichlorid und 3,2 Gew. % Phthalsäureanhydrid. Dies entspricht einer Rohausbeute von 95 % der Theorie.

Nach Feindestillation bei 110 bis 122°C und 1,4 bis 1,7 mbar werden als Hauptfraktion 659,9 g farbloses Destillat mit einem Produktgehalt von 97,4 Gew.% erhalten.

### Beispiel 2 (Vergleichsbeispiel; N,N-Dibutylformamid als Katalysator)

523,7 g (3,5 mol) Phthalsäureanhydrid und 2,75 g (0,02 mol) N,N-Dibutylformamid als Katalysator werden in 2203,4 ml Toluol vorgelegt, und die Mischung wird auf 70°C erwärmt. Bei dieser Temperatur werden über 6 Stunden 586,1 g (5,93 mol) Phosgen mit einer Einleitgeschwindigkeit von 97,7 g Phosgen pro Stunde und gleichzeitig 30,66 g N,N-Dibutylformamid gelöst in 218 ml Toluol kontinuierlich (5,1 g N,N-Dibutylformamid pro Stunde) zugegeben. Nach Ende der Dosierung von Phosgen und Katalysator wird noch 1,5 Stunden bei 70°C nachgerührt.

Zur Entfernung überschüssigen Phosgens werden bei 55°C und 60 mbar 2103,0 g Destillat abgenommen.

Als dunkles Öl bleibt das Rohprodukt zurück. Dieses enthält laut HPLC-Analytik 83,8 Gew.% Phthalsäuredichlorid, 2,4 Gew.% Phthalsäureanhydrid und laut GC-Analytik 0,9 Flächen% N,N-Dibutylcarbamoylchlorid. Bezogen auf das Produkt entspricht dies einer Rohausbeute von 97,8 % der Theorie.

Nach Feindestillation werden bei 85-113°C und 0,05 bis 0,2 mbar als Hauptfraktion 666,9 g violett gefärbtes Destillat mit einem Produktgehalt von 95,2 Gew.% und einem Gehalt an N,N-Dibutylcarbamoylchlorid von immer noch 0,9 Flächen% erhalten. Das entspricht einer Ausbeute von 89,3 % der Theorie.

### Beispiel 3 (erfindungsgemäß; N,N-Dibenzylformamid als Katalysator)

### Katalysatorpräparation:

Es werden 300,0 g (1,48 mol) Dibenzylamin und 74,7 g (1,62 mol) Ameisensäure in 384,2 ml Toluol bei 20°C vorgelegt. Diese Mischung wird 1 Stunde lang unter Rückfluss (95°C) erhitzt. Zur Entwässerung wird anschließend azeotropiert, bis sich kein Wasser mehr abscheidet (Gesamtmenge des abgenommenen Wassers: 30,9 g). Es werden 671,2 g toluolische Lösung des Katalysators N,N-Dibenzylformamid mit einem Gehalt von 49,5 % erhalten. Das entspricht einer Ausbeute von 99,9 % der Theorie. Die so erhaltene Katalysatorlösung (K1) wird im folgenden eingesetzt.

### Reaktion:

149,6 g (1,0 Mol) Phthalsäureanhydrid und 13,7 g (0,03 mol) der oben beschriebenen Katalysatorlösung (K1) werden in 620,9 ml Toluol vorgelegt, und die Mischung wird auf 75°C erwärmt. Bei dieser Temperatur werden über 5 Stunden 296,8 g (3,0 mol) Phosgen mit einer Einleitgeschwindigkeit von 59,4 g Phosgen pro Stunde und gleichzeitig 82,0 g der K1-Lösung kontinuierlich (8,1 g N,N-Dibenzylformamid 100%ig pro Stunde) zugegeben. Nach Ende der Dosierung von Phosgen und Katalysator wird noch 1,75 Stunden bei 75°C nachgerührt.

Zur Entfernung von überschüssigem Phosgen werden bei 55°C und 60 mbar 253,6 g Destillat abgenommen.

Als dunkles Öl bleibt das Rohprodukt zurück. Es enthält 69,7 Gew.% Phthalsäuredichlorid und 5,5 Gew. % Phthalsäureanhydrid. Das entspricht einer Rohausbeute von 87 % der Theorie.

Nach Feindestillation bei 100 bis 104°C und 0,3 mbar werden als Hauptfraktion 160,3 g farbloses Destillat mit einem Produktgehalt von 85,7 Gew.% erhalten. Das entspricht einer Ausbeute von 67,6 % der Theorie.

### Beispiel 4 (erfindungsgemäß, N,N-Dicyclohexylformamid als Katalysator)

22,4 g (0,15 Mol) Phthalsäureanhydrid und 0,16 g (0,8 mmol) N,N-Dicyclohexylformamid als Katalysator werden in 84,9 ml Toluol vorgelegt, und die Mischung wird auf 75°C erwärmt. Bei dieser Temperatur werden über 150 Minuten 22,6 g (0,23 mol) Phosgen mit einer Einleitgeschwindigkeit von 9,0 g Phosgen pro Stunde und gleichzeitig 1,73 g (0,01 mol) N,N-Dicyclohexylformamid gelöst in 19 ml Toluol gleichmäßig (0,7 g N,N-Dicyclohexylformamid 100%ig pro Stunde) zugegeben. Nach Ende der Dosierung von Phosgen und Katalysator wird noch 2,5 Stunden bei 75°C nachgerührt.

Zur Entfernung von überschüssigem Phosgen werden bei 60°C und 28 mbar 94 g Destillat abgenommen.

Als dunkles Öl bleibt das Rohprodukt zurück. Es enthält 90,8 Gew.% Dichlorid und 0,4 Gew.% Anhydrid. Das entspricht einer Rohausbeute von 99,9 % der Theorie.

Nach Feindestillation bei 98 bis 100°C und 0,1 bis 1,2 mbar werden als Hauptfraktion 28,3 g farbloses Destillat mit einem Produktgehalt von 95,2 Gew.% erhalten. Dies entspricht einer Ausbeute von 88,5 % der Theorie.

## Patentansprüche

1. Verfahren zur Herstellung von Phthalsäuredichlorid der Formel (I) 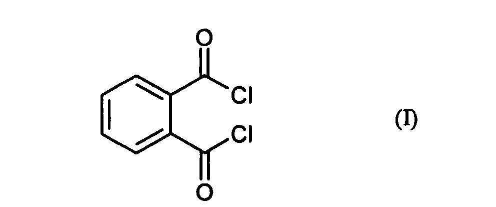 in dem man Phthalsäureanhydrid der Formel (II) 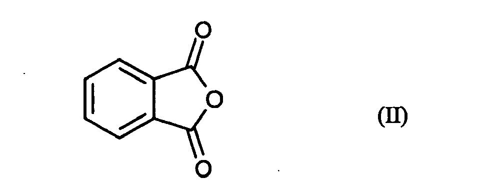 mit Phosgen in Gegenwart eines Katalysators umsetzt, **dadurch gekennzeichnet, dass** als Katalysator ein N,N-disubstituiertes Formamid der allgemeinen Formel (III) eingesetzt wird, 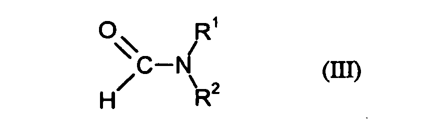 in welcher
R¹ und R² unabhängig voneinander für
- geradkettiges oder verzweigtes C₈-C₂₂-alkyl oder geradkettiges oder verzweigtes C₈-C₂₂-alkenyl,
- C₃-C₈-cycloalkyl,
- C₆-C₁₀-aryl oder
- C₇-C₁₂-arylalkyl
stehen, wobei für den Fall, dass beide Reste R¹ und R² unabhängig voneinander für geradkettiges oder verzweigtes C₈-C₂₂-alkyl oder geradkettiges oder verzweigtes C₈-C₂₂-alkenyl stehen, das Gesamtmolekulargewicht des N,N-disubstituierten Formamids mindestens 269 g/mol betragen muss.

2. Verfahren nach Anspruch 1, **dadurch gekennzeichnet, dass** ein N,N-disubstituiertes Formamid der Formel (III) eingesetzt wird, in der R¹ und R² beide für unabhängig voneinander für einen C₃-C₈-Cycloalkylrest, bevorzugt beide Reste R¹ und R² für denselben C₃-C₈-Cycloalkylrest und inbesondere beide Reste R¹ und R² für einen Cyclopentyl oder einen Cyclohexylrest stehen.

3. Verfahren nach einem oder mehreren der Ansprüche 1-2, **dadurch gekennzeichnet, dass** ein N,N-disubstituiertes Formamid der Formel (III) eingesetzt wird, in der R¹ und R² unabhängig voneinander für einen Phenyl- oder Naphthyl-Rest stehen können.

4. Verfahren nach einem oder mehreren der Ansprüche 1-3, **dadurch gekennzeichnet, dass** ein N,N-disubstituiertes Formamid der Formel (III) eingesetzt wird, in der R¹ und R² unabhängig voneinander für einen Benzyl-, Phenylethyl-, Phenylpropyl-, Naphthylmethyl- oder einen Naphthylethyl-Rest stehen können.

5. Verfahren nach Anspruch 1, **dadurch gekennzeichnet, dass** als N,N-disubstituierte Formamide der allgemeinen Formel (III)
- N,N-Dioctylformamid,
- N,N-Dinonylformamid,
- N,N-Didecylformamid,
- N,N-Diundecylformamid,
- N,N-Didodecylformamid,
- N,N-Ditridecylformamid,
- N,N-Ditetradecylformamid,
- N,N-Dipentadecylformamid,
- N,N-Dihexadecylformamid,
- N,N-Dioctadecylformamid,
- N,N-Dicyclopentylformamid;
- N,N-Dicyclohexylformamid oder
- N,N-Dibenzylformamid
verwendet werden.

6. Verfahren nach einem oder mehreren der Ansprüche 1-5, **dadurch gekennzeichnet, dass** man die N,N-disubstituierten Formamide der Formel (III) durch Umsetzung des entsprechenden Amins der Formel HNR¹R², wobei R¹ und R² die für die Formel (III) genannten Bedeutungen besitzen können, mit Ameisensäure herstellt und das dabei erhaltene Reaktionsgemisch unmittelbar für das Verfahren gemäß einem oder mehreren der Ansprüche 1-7 einsetzt.

7. Verfahren nach einem oder mehreren der Ansprüche 1-6, **dadurch gekennzeichnet, dass** man auf 1 mol Phthalsäureanhydrid der Formel (II) 1,0 mol bis 3,0 mol, vorzugsweise 1,4 mol bis 2,2 mol Phosgen einsetzt.

8. Verfahren nach einem oder mehreren der Ansprüche 1-7, **dadurch gekennzeichnet, dass** man bezogen auf 1 mol Phthalsäureanhydrid der Formel (II) insgesamt 0,01 - 0,20 mol, vorzugsweise 0,02 - 0,10 mol N,N-disubstituiertes Formamid der Formel (III) einsetzt.

9. Verfahren nach einem oder mehreren der Ansprüche 1-8, **dadurch gekennzeichnet, dass** Phthalsäureanhydrid gelöst in dem inerten Verdünnungsmittel vorgelegt und anschließend das Phosgen sowie das N,N-disubstituierte Formamid der Formel (III) jeweils unabhängig voneinander kontinuierlich oder "semi-kontinuierlich" eindosiert werden.

10. Verfahren nach Anspruch 9, **dadurch gekennzeichnet, dass** das N,N-disubstituierte Formamid der Formel (III) entweder ebenfalls gelöst in einem inerten Lösungsmittel zudosiert wird, welches bevorzugt identisch zum inerten Lösungsmittel für das Phthalsäureanhydrid ist, oder aber ohne inertes Lösungsmittel zudosiert wird, wofür das N,N-disubstituierte Formamid, sofern es als Feststoff vorliegt, zunächst aufgeschmolzen und dann als Schmelze zudosiert wird.

11. Verfahren nach einem oder mehreren der Ansprüche 1-10, **dadurch gekennzeichnet, dass** das Phthalsäureanhydrid in einem inerten Lösungsmittel zusammen mit der gesamten Menge oder einer Teilmenge des N,N-disubstituierten Formamids der Formel (III) vorgelegt, die Mischung danach auf die Reaktionstemperatur erhitzt und anschließend das Phosgen und gegebenenfalls das restliche N,N-disubstituierte Formamid der Formel (III) kontinuierlich oder "semi-kontinuierlich" eindosiert werden.

## Claims

1. Process for preparing phthaloyl chloride of the formula (I) 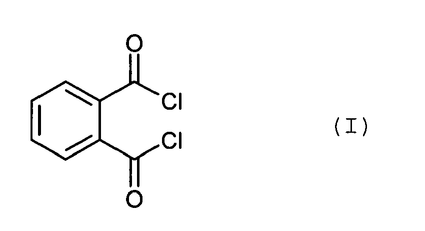 in which phthalic anhydride of the formula (II) 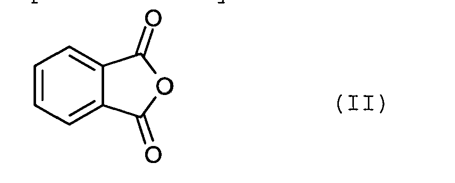 is reacted with phosgene in the presence of a catalyst, **characterized in that** the catalyst used is an N,N-disubstituted formamide of the general formula (III) 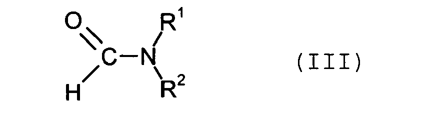 in which
R¹ and R² are each independently
- straight-chain or branched C₈-C₂₂-alkyl or straight-chain or branched C₈-C₂₂-alkenyl,
- C₃-C₈-cycloalkyl,
- C₆-C₁₀-aryl or
- C₇-C₁₂-arylalkyl
where, in the case that each R¹ and R² radical is independently straight-chain or branched C₈-C₂₂-alkyl or straight-chain or branched C₈-C₂₂-alkenyl, the total molecular weight of the N,N-disubstituted formamide has to be at least 269 g/mol.

2. Process according to Claim 1, **characterized in that** an N,N-disubstituted formamide of the formula (III) is used in which R¹ and R² are each independently a C₃-C₈-cycloalkyl radical, both R¹ and R² radicals are preferably each the same C₃-C₈-cycloalkyl radical, and both R¹ and R² radicals are in particular each a cyclopentyl or a cyclohexyl radical.

3. Process according to one or more of Claims 1-2, **characterized in that** an N,N-disubstituted formamide of the formula (III) is used in which R¹ and R² may each independently be a phenyl or naphthyl radical.

4. Process according to one or more of Claims 1-3, **characterized in that** an N,N-disubstituted formamide of the formula (III) is used in which R¹ and R² may each independently be a benzyl, phenylethyl, phenylpropyl, naphthylmethyl or a naphthylethyl radical.

5. Process according to Claim 1, **characterized in that** the N,N-disubstituted formamide of the general formula (III) used is
- N,N-dioctylformamide,
- N,N-dinonylformamide,
- N,N-didecylformamide,
- N,N-diundecylformamide,
- N,N-didodecylformamide,
- N,N-ditridecylformamide,
- N,N-ditetradecylformamide,
- N,N-dipentadecylformamide,
- N,N-dihexadecylformamide,
- N,N-dioctadecylformamide,
- N,N-dicyclopentylformamide,
- N,N-dicyclohexylformamide or
- N,N-dibenzylformamide.

6. Process according to one or more of Claims 1-5, **characterized in that** the N,N-disubstituted formamides of the formula (III) are prepared by reacting the corresponding amine of the formula HNR¹R² where R¹ and R² may each be as defined for the formula (III) with formic acid and using the resulting reaction mixture directly for the process according to one or more of Claims 1-5.

7. Process according to one or more of Claims 1-6, **characterized in that** 1.0 mol to 3.0 mol, preferably 1.4 mol to 2.2 mol, of phosgene are used for 1 mol of phthalic anhydride of the formula (II).

8. Process according to one or more of Claims 1-7, **characterized in that**, based on 1 mol of phthalic anhydride of the formula (II), a total of 0.01-0.20 mol, preferably 0.02-0.10 mol, of N,N-disubstituted formamide of the formula (III) is used.

9. Process according to one or more of Claims 1-8, **characterized in that** phthalic anhydride is initially charged dissolved in the inert diluent and the phosgene and the N,N-disubstituted formamide of the formula (III) are subsequently each independently metered in continuously or "semi-continuously".

10. Process according to Claim 9, **characterized in that** the N,N-disubstituted formamide of the formula (III) is either metered in likewise dissolved in an inert solvent which is preferably identical to the inert solvent for the phthalic anhydride or else is metered in without inert solvent, in which case the N,N-disubstituted formamide, when it is present in solid form, is initially melted and then metered in as a melt.

11. Process according to one or more of Claims 1-10, **characterized in that** the phthalic anhydride is initially charged in an inert solvent together with the entire amount or a portion of the N,N-disubstituted formamide of the formula (III), the mixture is then heated to the reaction temperature and the phosgene and any remaining N,N-disubstituted formamide of the formula (III) are subsequently metered in continuously or "semi-continuously".

## Revendications

1. Procédé pour la préparation de dichlorure de l'acide phtalique de formule (I) 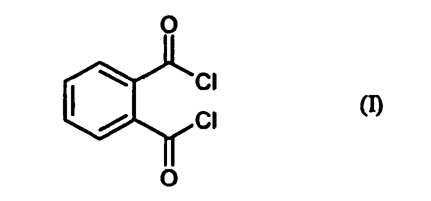 dans lequel on transforme de l'anhydride de l'acide phtalique de formule (II) 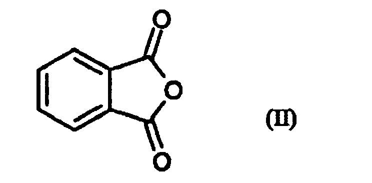 avec du phosgène en présence d'un catalyseur, **caractérisé**
**en ce qu'**on utilise comme catalyseur un formamide N,N-disubstitué de formule générale (III), 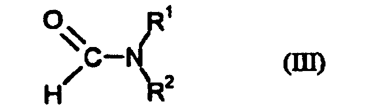 dans laquelle
R¹ et R² , indépendamment l'un de l'autre, représentent
- C₈-C₂₂-alkyle linéaire ou ramifié ou C₈-C₂₂-alcényle linéaire ou ramifié,
- C₃-C₈-cycloalkyle,
- C₆-C₁₀-aryle ou
- C₇-C₁₂-arylalkyle
où, dans le cas où les deux radicaux R¹ et R² représenteraient, indépendamment l'un de l'autre, un radical C₈-C₂₂-alkyle linéaire ou ramifié ou un radical C₈-C₂₂-alcényle linéaire ou ramifié, le poids moléculaire total du formamide N,N-disubstitué doit être d'au moins 269 g/mole.

2. Procédé selon la revendication 1, **caractérisé en ce qu'**on utilise un formamide N,N-disubstitué de formule (III), dans lequel R¹ et R² représentent tous les deux, indépendamment l'un de l'autre, un radical C₃-C₈-cycloalkyle, de préférence les deux radicaux R¹ et R² représentent le même radical C₃-C₈-cycloalkyle et en particulier les deux radicaux R¹ et R² représentent un radical cyclopentyle ou cyclohexyle.

3. Procédé selon l'une ou plusieurs des revendications 1-2, **caractérisé en ce qu'**on utilise un formamide N,N-disubstitué de formule (III), dans lequel R¹ et R² peuvent représenter indépendamment l'un de l'autre un radical phényle ou naphtyle.

4. Procédé selon l'une ou plusieurs des revendications 1-3, **caractérisé en ce qu'**on utilise un formamide N,N-disubstitué de formule (III), dans lequel R¹ et R² peuvent représenter indépendamment l'un de l'autre un radical benzyle, phényléthyle, phénylpropyle, naphtylméthyle ou naphtyléthyle.

5. Procédé selon la revendication 1, **caractérisé en ce qu'**on utilise comme formamide N,N-disubstitué de formule générale (III)
- du N,N-dioctylformamide,
- du N,N-dinonylformamide,
- du N,N-didécylformamide,
- du N,N-di-undécylformamide,
- du N,N-didodécylformamide,
- du N,N-ditridécylformamide,
- du N,N-ditétradécylformamide,
- du N,N-dipentadécylformamide,
- du N,N-dihexadécylformamide,
- du N,N-dioctadécylformamide,
- du N,N-dicyclopentylformamide,
- du N,N-dicyclohexylformamide ou
- du N,N-dibenzylformamide.

6. Procédé selon l'une ou plusieurs des revendications 1-5, **caractérisé en ce qu'**on prépare les formamides N,N-disubstitués de formule (III) par transformation de l'amine correspondante de formule HNR¹R², où R¹ et R² peuvent présenter les significations mentionnées pour la formule (III), avec de l'acide formique et on utilise le mélange réactionnel obtenu directement pour le procédé selon l'une ou plusieurs des revendications 1-5.

7. Procédé selon l'une ou plusieurs des revendications 1-6, **caractérisé en ce qu'**on utilise pour 1 mole d'anhydride de l'acide phtalique de formule (II) 1,0 mole à 3,0 moles, de préférence 1,4 mole à 2,2 moles de phosgène.

8. Procédé selon l'une ou plusieurs des revendications 1-7, **caractérisé en ce qu'**on utilise, par rapport à 1 mole d'anhydride de l'acide phtalique de formule (II) au total 0,01-0,20 mole, de préférence 0,02-0,10 mole de formamide N,N-disubstitué de formule (III).

9. Procédé selon l'une ou plusieurs des revendications 1-8, **caractérisé en ce qu'**on dispose au préalable l'anhydride de l'acide phtalique sous forme dissoute dans un diluant inerte puis on introduit en dosant le phosgène ainsi que le formamide N,N-disubstitué de formule (III) à chaque fois indépendamment l'un de l'autre, en continu ou de manière "semi-continue".

10. Procédé selon la revendication 9, **caractérisé en ce que** le formamide N,N-disubstitué de formule (III) est dosé soit également sous forme dissoute dans un solvant inerte, qui est de préférence identique au solvant inerte pour l'anhydride de l'acide phtalique, soit également sans solvant inerte, où le formamide N,N-disubstitué, pour autant qu'il se trouve sous forme de solide, est d'abord fondu puis dosé sous forme de masse fondue.

11. Procédé selon l'une ou plusieurs des revendications 1-10, **caractérisé en ce que** l'anhydride de l'acide phtalique est disposé au préalable dans un solvant inerte, ensemble avec la quantité totale ou une quantité partielle du formamide N,N-disubstitué de formule (III), le mélange est ensuite chauffé à la température de réaction puis le phosgène et le cas échéant le reste du formamide N,N-disubstitué de formule (III) est introduit en dosant en continu ou de manière "semi-continue".
